(19) Europäisches Patentamt
European Patent Office
Office européen des brevets

(11) Publication number: **0 248 621 B1**

(12) **EUROPEAN PATENT SPECIFICATION**

(45) Date of publication of patent specification: **26.08.92**

(51) Int. Cl.$^5$: **G01N 33/532**, G01N 33/546, G01N 33/68, G01N 33/574

(21) Application number: **87304826.8**

(22) Date of filing: **01.06.87**

(54) **Reagent for immunoassay.**

(30) Priority: **31.05.86 JP 126381/86**

(43) Date of publication of application: **09.12.87 Bulletin 87/50**

(45) Publication of the grant of the patent: **26.08.92 Bulletin 92/35**

(84) Designated Contracting States: **DE FR GB IT**

(56) References cited:
**EP-A- 0 141 620**
**EP-A- 0 144 084**
**GB-A- 2 052 059**
**GB-A- 2 079 936**
**US-A- 4 444 879**

(73) Proprietor: **KABUSHIKI KAISHA TOSHIBA**
**72, Horikawa-cho Saiwai-ku**
**Kawasaki-shi Kanagawa-ken 210(JP)**

(72) Inventor: **Hatoh, Masako, c/o Patent Division**
**Kabushiki Kaisha Toshiba, 1-1 Shibaura 1-chome**
**Minato-ku, Tokyo 105(JP)**
Inventor: **Ishimori, Yoshio, c/o Patent Division**
**Kabushiki Kaisha Toshiba, 1-1 Shibaura 1-chome**
**Minato-ku, Tokyo 105(JP)**
Inventor: **Koyama, Masao, c/o Patent Division**
**Kabushiki Kaisha Toshiba, 1-1 Shibaura 1-chome**
**Minato-ku, Tokyo 105(JP)**

(74) Representative: **Freed, Arthur Woolf et al**
**MARKS & CLERK 57-60 Lincoln's Inn Fields**
**London WC2A 3LS(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to reagents for immunoassays useful for quantitatively analyzing a sepcific antigen or antibody in a sample.

Radio immunoassays (referred to as RIA hereinafter) can be used for the quantitative analysis of a specific antigen or antibody in a sample, e.g., a biological sample. However RIA involves the use of a radioactive element which results in disadvantages. Problems such as the necessity of installing instruments for exclusive use and of performing the analytical work by a licensed operator, and careful disposal of wastes and the like render RIA problematic.

Immunoelectrophoresis is also known as a method for performing these analyses. However, immunoelectrophoresis suffers from, in addition to long times required for detection, low sensitivity. Immunoelectrophoresis has the problem that it cannot be used if the content of material to be detected is extremely low.

In view of the above, the inventors of the present invention have disclosed in Japanese Patent No. 60-117159, new liposome reagents which can be used in immunoassays. The liposome reagents have hydrophilic antibodies or antigens immobilized on the surface of the liposome. These new liposome reagents are added to a sample which contains an antibody or antigen, and a complement is added to the sample separately. The liposomes are destroyed by an antigen-antibody reaction and an accompanying action of the complement activated by the reaction, and a marker material (e.g., a fluorescent compound) enclosed in the liposomes is released. Because a certain correlation exists between the released amount of marker material and the amount of the material to be detected in the sample, the material to be detected can be analyzed quantitatively through a quantitative analysis of the released marker material. The quantitative analysis of the released marker material is achieved using a predetermined analytical method (e.g., fluorometric analysis). The use of such a reagent does not lead to the problems found with RIA so that these new reagents simplify of immunoassays.

However these new liposome reagents suffer from the disadvantage that if an undiluted serum sample or an undiluted protein-containing sample is analyzed with this new liposome reagent, reactions other than the antigen-antibody reaction can take place. This can lead to difficulties in performing the accurate analysis of an undiluted solution containing a material to be detected. For this reason, serum or protein-containing samples to be analyzed had to be diluted.

For instance, to analyze $\alpha$-fetoprotein (referred to as AFP hereinafter) in a human serum sample using a reagent obtained by immobilizing anti-human $\alpha$-fetoprotein antibodies (referred to as anti-human AFP antibodies hereinafter) on liposomes, the human serum sample has to be diluted more than 100-fold to eliminate the influence of nonspecific reactions. However, since the serum of normal human beings contains less than $10^{-8}$ g/mℓ of AFP, the dilution of more than 100 times of the serum thus requires measurement (for instance, fluorometric analysis) of an AFT concentration of less than $10^{-10}$ g/mℓ. This necessity of diluting the sample and thus lowering the concentration of the material to be analyzed leads to the problem that accurate quantitative analysis is difficult.

There is therefore a strongly felt need for a new method and/or a new reagent permitting facile and accurate immunoassays.

Accordingly, it is an object of this invention to provide a new reagent useful for accurate immunoassays.

It is another object of this invention to provide a new reagent useful for the accurate immunoassay of materials present in a sample at very low concentrations.

It is another object of this invention to provide a new, safe reagent useful for immunoassays.

The inventors have now surprisingly discovered a new immunoassay reagent which satisfies all of these objects and other objects which will become apparent from the description of the invention given hereinbelow.

In this invention, the inventors have discovered that functional groups used to immobilize the antibodies, antigens, or parts of antibodies onto the liposome which remain after immobilizing the antibody or a part of the antibody, or antigen on the liposome, obstruct precise measurement and analysis through their reaction with proteins and minute amount of chemical substances that exist in samples. The present invention solves this heretofore unrecognized problem and provides a new and better reagent for immunoassays.

The reagent for immunoassays of this invention is defined in claim 1 below, and the method according to the invention for assaying is defined in claim 15 below.

This invention can be more fully understood from the following detailed description of a preferred embodiment when taken in conjunction with the accompanying drawings, in which:

Figs. 1A and 1B are schematic diagrams which show the blocking behaviour for a functional group for immobilization that is left remained on a liposome, with a substance which contains an SH group

(cysteine); and

Fig. 2 is a calibration curve of the liposomes of the present invention for human AFP.

To block the immobilizing functional group which immobilizes the antibody or a part of the antibody, or the antigen on the liposome surface, one chemically modifies the functional group with a blocking agent. This blocking agent can be a chemical substance which contains an SH group or a chemical substance which contains an amino group. To be more specific, the immobilizing functional groups remaining on the liposome are substituted by causing the functional group to react under appropriate conditions, with an SH-group-containing substance such as cysteine or dithiothreitol that are examples of water-soluble reducing substances or with an amino-group-containing substance such as glycine or TRIS.

In the reagent for immunoassays of the present invention, the liposome is composed of at least one phospholipid, or one glycolipid. The liposome has a monolayered or multi-layered microcapsular form. Moreover, 10 to 500 molar % of cholesterol may be added, if necessary, to the phospholipid or glycolipid to obtain more stable liposomes. The number of carbon atoms in the carbon chain of the fatty acid in the phospolipid or glycolipid is preferred to be 12 to 18, and it is more desirable that the number is even.

In the reagent for immunoassays of the present invention, the marker material which is enclosed in the liposome may be a material which is hydrophilic and can be analyzed quantitatively when released to the outside of the liposomes. Examples of such marker materials include, for example:

fluorescent materials such as carboxyfluorescein which emit no fluorescence due to self-quenching at a high concentration but gives-off a very intensive fluorescence at a low concentration ($10^{-3}$ M or less);

luminous substances such as luminol or luciferin which emit light owing to an oxidative reaction outside of the liposomes;

absorptive compounds (water-soluble pigments and others) and which posses specific absorption bands in the visible or ultraviolet region;

saccharides such as glucose or sucrose which are decomposed by the action of an oxidase and bring about oxygen consumption or production of hydrogen peroxide;

relatively large ionic compounds such as tetrapentyl ammonium;

coenzymes such as nicotinamideadenine-dinucleotide (NAD);

radical compounds such as methylviologen;

and so on.

The compounds mentioned above are suitably selected by taking into consideration factors such as a method and sensitivity of detection and a stability of liposomes.

In the reagent for immunoassays of the present invention, the antibody or a part of the antibody or the antigen to be immobilized on the liposomes can be a protein, a peptide, or a glycoprotein. It can be arbitrarily selected in accordance with the measurement which is to be made. These materials can be chosen from among, for example, immunoglobulins such as IgG, IgE, IgD, IgA, and IgM; enzymes such as transaminase, lactate dehydrogenase, and creatinephosphokinase, tumor markers such as AFP and carcinoembryonic antigen (CEA); peptide hormones such as insulin and growth hormone; and low molecular substances such as various kinds of drugs.

In the reagent for immunoassays of the present invention, various materials can be appropriately used as cross-linking agents to immobilize the antibody, part of the antibody or the antigen onto the liposome. These materials are reacted with lipid molecules to introduce an immobilizing functional group onto the liposomes. Materials can also be used which act as a cross-linking agent for introducing, if necessary, a cross-linking group to an antibody or a part of the antibody, or to an antigen. These materials include materials which lead, for instance, to the following reaction schemes.

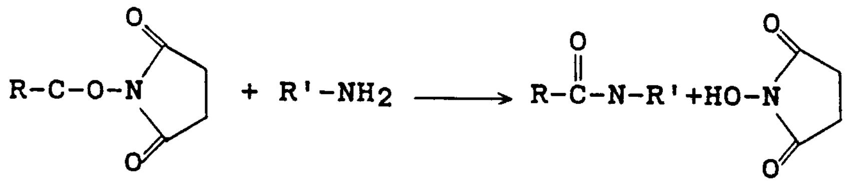

(A N-Hydroxysuccinimide Ester Reaction Scheme) (1)

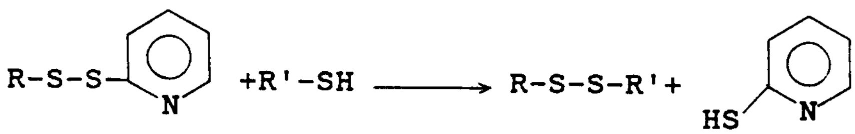

(A Pyridyl Disulfide Reaction Scheme) (2)

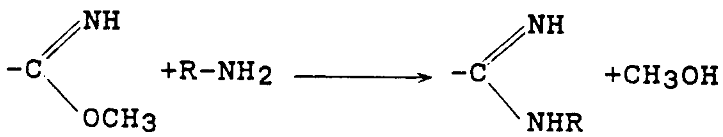

(An Imidoester Reaction Scheme) (3)

Thus, such cross-linking agents include disuccinimidyl suberate (DSS), 3,3′-dithiobis-(sulfosuccinimidylpropionate) (DTSSP), ethylene glycobis(sulfosuccinimidylsuccinate) (Sulf-EGS), and other cross-linking agents that are represented by the reaction based on Eq. (1), N-succinimidyl 3-(2-pyridyldithio)propionate (SPDP), and others represented by Eq. (2), and dimethyl adipimidate•2HCℓ (DMA), dimethyl 3,3′-dithiobispropionimidate•2HCℓ (DTBP), and others represented by Eq. (3).

Of these, SPDP which is represented by the following formula

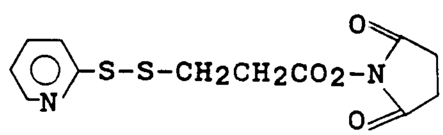

is a heterobifunctional reagent used for thiolation that proceeds under moderate conditions and for formation of intermolecular (cross-linked) conjugates.

In the reagent for immunoassays in accordance with the present invention which make use of such cross-linking agents as in the above, an antigen, or an antibody or a part of the antibody which is immobilized on the liposomes is bonded to the liposomes without having a ring structure, such as pyridyl group and N-substituted succinimide group, interposed between the liposome. Preferred forms of such bondings include -S-S- bonds and -C-N- bonds.

The reagents for immunoassays of the present invention can be prepared by the following method. First, a functional lipid is obtained by functionalizing lipid molecules through the reaction of a desired lipid with a cross-linking agent in a solvent. The functional group in the functional lipid acts as an immobilizing functional group which immobilizes an antibody or a part of the antibody, or an antigen onto the liposomes. Next, the functional lipid obtained, and if necessary, an adequate amount of cholesterol and another lipid, are taken into a flask to be dissolved in and mixed with a solvent. The solvent is then removed under reduced pressure to dry the product. As a result, a thin lipid membrane is formed on the inner surface of the flask wall. Subsequently, an aqueous solution containing a marker material is added to the content of the flask, and a liposome suspension is obtained by tightly sealing and shaking the flask.

On the other hand, after introducing, if necessary a cross-linking group through reaction with a cross-linking agent, to the antibody, a part of the antibody or the antigen to be immobilized on the liposomes, the product is modified by treating with a reducing agent depending upon need.

Then, the antibody or a part of the antibody or the antigen is immobilized onto the liposomes by causing the liposome suspension to react with the antibody or a part of the antibody or the antigen in an appropriate buffer solution.

When the liposomes are bonded with the antigen, antibody or a part of the antibody by the intermediacy of a functional group which reacts with amino groups such as succinimide group, imide ester group, aldehyde group, and acidic chloride group, the functional groups remaining on the surface of the liposomes are blocked by causing the group to react under suitable conditions with an amino acid such as glycine, serine, or TRIS, or with substances containing amino group such as proteins like albumins or enzymes. Further, when the bonding is achieved by the intermediacy of a functional group such as dithiopyridyl group which reacts with SH group, the pyridyl group that is considered to be remaining on the liposome surface may be blocked, as shown in Fig. 1, by causing the group to react with a water-soluble reducing substance containing an SH group, such as cysteine, dithiothreitol, or mercaptoethanol.

In the above, conditions suitable for the blocking imply conditions on pH, ionic strength, concentration, time, temperature, osmotic pressure, and so forth which do not disturb the antibody or the like that is immobilized on the liposome surface. An example of such a condition is to wash the liposomes with a HEPES-saline buffer solution (pH 7.5) containing cysteine which is a substance containing SH groups.

As a result of such blocking of the immobilizing functional group, the immobilizing functional group remaining on the liposomes can be eliminated so that the reagent for immunoassay in the present invention will not participate in nonspecific reactions of the functional group with proteins or minute amount of other chemical substances contained in the sample being analyzed. The possibility of obstructing precise measurement and analysis, due to breakage of the liposomes is thus avoided.

The reagent for immunoassay of the present invention can be used in the following manner. Namely, first, the reagent for immunoassay of the present invention is added to a sample that contains a material to be detected, and a complement is added separately. In this stage, a method may be adopted in which a second antibody is added to interpose the material to be detected (sandwich assay). As a result, the liposomes are broken due to the antigen-antibody reaction between the material to be detected and the antigen, antibody or a part of the antibody that is immobilized on the liposomes, and an accompanying action of the complement activated by the reaction. Thereby the marker material (for instance, a fluorescent compound) enclosed in the liposomes is released. Since there exists a correlation between the amount of the marker material released and the amount of the material to be detected in the sample, the material to be detected can be analyzed quantitatively, by analyzing the amount of marker material released using a suitable method of analysis (for instance, fluorometric analysis).

In practice, quantitative analysis for the material to be detected is carried out by preparing beforehand a calibration curve using the material to be detected at known concentrations. One then measures from this calibration curve the amount of the marker material released under identical conditions in the sample material and thus the unknown concentration of material being analyzed is determined.

The length of time which is required for a thorough reaction between the reagent for immunoassay and the material to be detected, differs depending upon the kind of the material to be detected, characteristics of the liposomes reaction conditions, and further, upon the kind, purity, form of immobilization, and so forth of the antigen, antibody or part of the antibody which is immobilized on the liposomes. For this reason, it is desirable for each individual case to set an optimum reaction time between the said reagent and the material to be detected by carrying out a preliminary measurements using samples containing the same kind of material as the material to be detected at known concentrations.

Materials that can be analyzed quantitatively by the reagent for immunoassays of the present invention cover a wide range of different materials. These include tumor markers ($\alpha$-fetoprotein (AFP), basic fetoprotein (BFP), carcinoembryonic antigen (CEA), pancreatic oncofetal antigen (POA), and so on), immunoglobulins (IgG, IgE, IgD, IgA, IgM, and so on), peptide hormones (insulin, growth hormone, and so on), drugs, amino acids, and so forth.

With the reagents of the present invention, residual immobilization functional groups left on the liposomes after immobilization of the antigen, antibody or a part of the antibody on the liposomes, are blocked completely. Consequently, nonspecific reactions of the functional group with proteins or minute amount of chemical substance in, for instance, the serum that serves as a sample, do not occur. Therefore, there is no need to dilute the sample to obtain a precise quantitative analysis. This greatly simplifies the operation of the analysis while at the same time increasing the applications of these reagents.

Other features of the invention will become apparent in the course of the following descriptions of exemplary embodiments which are given for illustration of the invention.

Example 1: Measurement of Human AFP

Of the reagents used in these example, dipalmitoylphosphatidyl ethanolamine (DPPE), dipalmitoylphosphatidyl choline (DPPC), cholesterol, and dithiothreitol (DTT) were manufactured by Sigma Inc., N-succinimidyl 3-(2-pyridyldithio) propionate (SPDP) and Sephadex G-25 Fine were manufactured by Pharmasia Fine Chemicals Inc. Other reagents were commercially available (special grade) and were used without further purificaion. The water used was exclusively ion-exchanged water.

(1) Functional phospholipid: Preparation of Dithiopyridyl Propionic Acid Amide DPPE (DTP-DPPE):

A conical flask equipped with a stopper was charged with 70 mg of DPPE dissolved in 25 mℓ of a chloroform and methanol mixture (in the ratio of 5:1 (v/v)). After addition of 60 μℓ of triethylamine and 50 mg of SPDP, and flushing the flask with nitrogen, the system was reacted for one hour at room temperature, and dithiopyridyl propionic acid amide (DTP) was introduced. The solvent was then removed with a rotary evaporator. The resulting dried material was dissolved in a mixture of chloroform and methanol (10:1 (v/v)), and the solution obtained was refined by passing through a silica gel column, and the DTP-DPPE fraction was collected. The obtained fraction was concentrated to about 5 mℓ by rotary evaporator. The yield of DTP-DPPE was in the range of 80 to 95%. The obtained solution was stored at -20 °C under a nitrogen gas atmosphere.

With these reactions, the dithiopyridyl group that was introduced to DPPE becomes a functional group for immobilization.

(2) Preparation of Liposomes:

All lipids used were dissolved in chloroform or a mixture of chloroform and methanol (2:1 (v/v)).

In a 10 mℓ eggplant-shaped flask, were placed 200 μℓ of 5 mM DPPC, 100 μℓ of 10 mM cholesterol, and 50 μℓ of 1 mM DTP-DPPE (functional phospholipid obtained in item (1) above). 2 mL of chloroform was then added and the content of the flask was mixed thoroughly. The solvent was then removed in a water bath of about 40 °C by means of a rotary evaporator. An additional 2 mℓ of chloroform was added and after a thorough mixing, the solvent was removed again using the rotary evaporator. Repeating the operation several times, a thin lipid membrane was formed on the wall surface of the flask. The flask was then transferred into a desicator, and suction was carried out for about one hour with a vacuum pump to completely remove the solvent.

Next, 100 μℓ of 0.2 M carboxyfluorescein (manufactured by Eastman Kodak Co., pH 7.4; referred to as CF hereinafter) were added thereto, and after flashing the interior of the flask with nitrogen, the flask was sealed tightly and immersed in a water bath at about 60 °C for about one minute. Then, the flask was shaken vigorously with a vortex mixer until the thin lipid membrane disappeared from the wall surface. A liposome suspension was prepared by the above operation. After adding a small amount of 0.01M HEPES buffer solution (containing 0.85% of NaCℓ, pH 7.45; referred to as HBS hereinafter) to the liposome suspension, the entire content of the flask was transferred to a centrifuge tube, and a centrifugal operation at 15,000 rpm and 4 °C for 20 minutes was repeated for several time. Finally, the product was suspended in 1 mℓ of HBS.

(3) Modification of an Anti-Human AFP Antibody:

After diluting 2 mℓ of an anti-human AFP antibody to 2 mg/mℓ with HBS and adding thereto 10 μℓ of 10 mM SPDP ethanol solution, dithiopyridyl group was introduced to the anti-human AFP antibody by bringing the above mixture into reaction at room temperature for 30 minutes. Next the reaction membrane was refined by removing the unreacted SPDP by gel filtration using a Sephadex G-25 Fine column (gel volume of about 15 mℓ) that was equilibrated in advance with a 0.1 M acetic acid buffer solution (containing 0.85% of Nacℓ, pH 4.5), and the protein fraction alone was collected.

Subsequently, after addition of about 20 mg of DTT to the fraction and flashing the reaction vessel with nitrogen, the mixture was brought to a reaction at room temperature for 20 minutes to modify the dithiopyridyl group by replacing it with the SH group. The protein fraction alone was then collected by refining the mixture, through removal of the unreacted DTT by gel filtration using a Sephadex G-25 Fine column equilibrated with HBS.

(4) Preparation of Liposomes with Immobilized Anti-Human AFP Antibody:

A liposome sediment obtained by a centrifuging, at 4 °C and 15,000 rpm for 20 minutes, 1 mℓ of the liposome suspension obtained in item (2) above, was mixed with 2 mℓ of the modified anti-human AFP antibody solution with 1 mg protein/mℓ obtained in item (3). After flashing the reaction vessel with tightly

sealing the same, the mixture was allowed to react overnight at 20°C while being shaken gently. The unreacted antibody was then removed by washing with HBS.

(5) Blocking of the Functional for Immobilization:

Sediment of the liposomes with immobilized anti-human AFP antibody obtained in item (4) above was mixed with 2 mℓ of 1 mM cysteine solution diluted with HBS. After flashing the reaction vessel with nitrogen and tightly sealing the same, the mixture was reacted overnight at 20°C with gentle shaking. Subsequently, the product was washed successively with HBS and gelatin-Veronal buffer solution (referred to as $GVB^-$hereinafter) to remove unreacted cysteine.

As a result, the immobilizing functional group (dithiopyridyl group) that remained on the liposome surface was blocked by the reaction of the immobilizing functional group with cysteine.

Two milliliters of $GVB^-$ and 20 μℓ of 10% $NaN_3$ were added to the liposome reagent obtained in the above manner. After a thorough stirring and a nitrogen replacement, the mixture was kept at 4°C.

(6) Preparation of a Calibration Curve for Human AFP Concentration by the Use of a Liposome Reagent with Immobilized Anti-human AFP Antibody:

A calibration curve was prepared by quantitatively analyzing human AFP concentration in the following way by means of a sandwich assay which utilizes an isolated anti-human AFP antibody of rabbit.

To 25 μℓ of a human AFP solution having a concentration that is varied in the range of 0.01 to 1,000 mg/mℓ, 5 μℓ of a liposome reagent which was diluted 30-fold in advance with $GVB^+$ (obtained from $GVB^-$ by the addition of 0.5 mM of $MgCℓ_2$ aqueous solution and 0.15 mM of $CaCℓ_2$ aqueous solution) was added. The mixture was left to react at 37°C for 10 minutes. Next, 25 μℓ of a rabbit anti-human AFP antibody (manufactured by Dako Inc.) which was diluted 200-fold in advance with $GVB^+$ were added thereto, and the mixture was left to react at 37°C for 5 minutes. Subsequently, 25μℓ of a complement (guinea pig serum 5 $CH_{50}$) was added, and the mixture was left to react at 37°C for 30 minutes.

In the above, $CH_{50}$ represents the value which is determined based on Mayer's method. Namely, first, red blood cells of sheep sensitized with the hemolysin at an optimum concentration and a serum are reacted in a buffer solution under optimum conditions for 60 minutes at 37°C. The sensitized red blood cells of sheep undergo hemolysis by the action of a complement so that the value of the complement can be determined by colorimetrically testing the degree of hemolysis using a radiation with wavelength 541 nm. According to the Mayer's method, one unit of a complement (1 $CH_{50}$ unit) is defined as the amount of the complement required for the hemolysis of 50% of $5 \times 10^8$ sheep red blood cells that are sensitized with hemolysin of an optimum concentration, at 37°C and during 60 minutes, in a reaction system of 7.5 mℓ volume.

Subsequently, the reaction was interrupted by the addition of 100 μℓ of 0.01 M EDTA-Veronal buffer solution, and the amount of CF was measured for human AFP solutions of various concentrations, using MTP-32 fluorospectrometer (made by Corona Electric Co., Ltd.) under the conditions of excitation wavelength 490 nm and fluorescence wavelength 520 nm.

Based on these measurements, relative fluorescence intensity was calculated according to the following equation, in order to eliminate the influence on the complement of the stability of liposomes with immobilized antibodies.

$$\text{Relative fluorescence intensity} = \frac{Fe-Fo}{Fa-Fo} \times 100.$$

In the above expression, Fe is the fluorescence intensity actually measured, Fo is the fluorescence intensity when antigens are removed (namely, when liposomes are not destroyed at all), and Fa is the fluorescence intensity when all the liposomes are destroyed by the addition of deionized water. In the calculation, fluorescence intensities of CF solutions with $10^{-7}$ M and $10^{-8}$ M were used as the standard values.

In this manner, the calibration curve shown in Fig. 2 which shows the relation between the human AFP concentration and the relative fluorescence intensity was obtained.

(7) Measurements of Human AFP Concentration:

Using the calibration curve obtained beforehand in item (6), human AFP concentration was measured on serum sample (No. 1 through No. 10) of 10 patients. The result of the measurements is summarized in Table 1. In table 1, Reference Example represents the measured value by RIA.

Comparative Example 1

A reagent for immunoassay was prepared for Comparative Example by carrying out identical procedures, item (1) through item (7), as in Example 1, except for the omission of the blocking procedure of the immobilized functional group. A calibration curve for the human AFP concentration was obtained using the reagent prepared as in the above, and the human AFP concentration was measured for the identical samples in Example 1. The result of the measurement is also included in Table 1.

As may be clear from Table 1, with the immunoassay reagent for Example 1, measurements that agree very well with the measurements by RIA were obtained. In contrast, with the immunoassay reagent of Comparative Example 1, it was impossible to take measurement for most of the cases. This is due to the fact that with the immunoassay reagent of Comparative Example 1, there were occurring nonspecific reactions between the functional group and the substances in the samples that are other than the material to be detected.

Table 1

| Sample No. | Measured Values of AFP (ng/mℓ) | | |
|---|---|---|---|
| | Reference Example | Example 1 | Comparative Example 1 |
| 1 | 760 | 758 | 800 |
| 2 | 261 | 259 | 532 |
| 3 | 25 | 25 | 496 |
| 4 | 58 | 60 | 59 |
| 5 | 314 | 315 | 764 |
| 6 | 60 | 60 | 533 |
| 7 | 34 | 35 | 561 |
| 8 | 11 | 8 | 507 |
| 9 | 1110 | 1110 | 1110 |
| 10 | 650 | 650 | 911 |

Example 2: Measurement of Human AFP

Liposomes were obtained in a manner analogous to Example 1 except for the blocking of the immobilizing functional group in item (5) of Example 1 which was carried out in the following way.

The mixture of sediment of liposomes with immobilized anti-human AFP antibodies, and 2 mℓ of 3 mM cysteine solution which was diluted with 0.01 M phosphate buffer solution (pH 7.7) was sealed tightly, and was brought to a reaction at 20°C for one hour while shaking gently. Subsequently, unreacted cysteine was removed by washing with gelatin-Veronal buffer solution. As a result, the immobilizing functional group (dithiopyridyl group) remaining on the liposome surface was blocked by reaction with cysteine.

After adding 2 mℓ of GVB- and 20 μℓ of 10% $NaN_3$ to the liposome reagent obtained in the above manner and giving a thorough stirring, the reaction vessel was flashed with nitrogen, and the mixture was stored at 4°C.

Next, after obtaining a calibration curve using the liposomes obtained as in the above, in a manner

analogous to Example 1, human AFP concentration was measured on serum samples (No. 11 through No. 20) of 10 patients. The result of the measurement is shown in Table 2. In Table 2, the values for Reference Example are those obtained by RIA.

Comparative Example 2

By preparing an immunoassay reagent by carrying out procedures identical to those of Example 2 except for the omission of the blocking of the immobilizing functional group that corresponds to the procedure in item (5) of Example 1, and obtaining a calibration curve for human AFP concentration, the human AFP concentration was measured for the samples identical to those of Example 2. The result is shown in Table 2.

As may be seen from Table 2, use of the immunoassay reagent for Example 2 leads to measured values that are in good agreement with the values obtained by RIA. In contrast, in the case of the immunoassay reagent of Comparative Example 2, it was impossible to take measurement for most of the cases. This is due to the fact that in the case of the immunoassay reagent of Comparative Example 2, there are generated nonspecific reactions between the functional group and the substances in the sample solutions that are other than the material to be detected.

**Table 2**

| Sample No. | Measured Values of AFP (ng/mℓ) | | |
|---|---|---|---|
| | Reference Example | Example 2 | Comparative Example 2 |
| 11 | 312 | 312 | 1566 |
| 12 | 63 | 63 | 1014 |
| 13 | 110 | 111 | 2007 |
| 14 | 577 | 577 | 1864 |
| 15 | 2350 | 2346 | 2562 |
| 16 | 4411 | 4410 | 5667 |
| 17 | 26 | 25 | 3378 |
| 18 | 396 | 395 | 6520 |
| 19 | 532 | 533 | 3270 |
| 20 | 16 | 19 | 3332 |

Example 3: Measurement of Human CEA

(1) Preparation of Functional Phospholipid and Preparation of Liposome:

A liposome suspension was prepared by carrying out the procedures that are identical to those of items (1) and (2) of Example 1.

(2) Modification of a Monoclonal Anti-Human CEA Antibody:

Using a monoclonal anti-human antibody, the SH group was introduced by a procedure similar to item (3) of Example 1.

(3) Preparation of Liposomes with an Immobilized Monoclonal Anti-Human CEA Antibody:

The liposome sediment obtained by centrifuging 1 mℓ of liposome suspension obtained by (1) above at 4°C and 15,000 rpm for 20 minutes, was mixed with 2 mℓ of the modified monoclonal anti-human CEA antibody solution (1 mg of proteins/mℓ). After flashing the reaction vessel with nitrogen and tight sealing, the mixture was reacted for 8 hours at 20°C with gentle shaking.

Next, cysteine was gently added to the obtained mixture so as to have a final concentration of 1.5 mM, and the reaction vessel was flashed with nitrogen. Then, the system was tightly sealed, and the immobilizing functional group that was remaining on the liposomes was blocked by causing the system to react overnight at 20°C while shaking the system gently. Following this, unreacted anti-human CEA antibody and cysteine were removed by successive washing with HBS and $GVB^-$.

After adding 2 mℓ of $GVB^-$ and 20 μℓ of 10% $NaN_3$ to the liposome reagent obtained as in the above, stirring thoroughly, flashing the reaction vessel with nitrogen and the system was stored at 4°C.

(4) Measurement of human CEA Concentration by a Liposome Reagent with Immobilized Monoclonal Anti-human CEA Antibody:

By means of the sandwich assay which uses a liberated rabbit anti-human CEA antibody, a calibration curve was obtained by examining the relation between the human CEA concentration and the relative fluorescence intensity similarly to (6) to (7) of Example 1. Using the calibration curve, measurement of the human CEA concentration was taken of the serum samples (No. 21 through No. 30) of 10 patients. The result is shown in Table 3. The values for Reference Example in Table 3 are the values obtained by RIA.

Comparative Example 3

Reagents for immunoassay were prepared by carrying out procedures identical to those of items (1) to (4) of Example 3 above, except for the omission of the blocking procedure of the immobilizing functional group described in (3). A calibration curve for human CEA concentration was obtained, and the human CEA concentration was measured for the samples identical to those of Example 3. The result is shown in Table 3.

As may be seen from Table 3, the immunoassay reagents of Example 3 lead to measured values that agree very well with the values obtained by RIA. In contrast, in the case of the immunoassay reagents of Comparative Example 3, it was not possible to take measurement for most of the samples. This is due to the fact that the immunoassay reagent of Comparative Example 3 generates nonspecific reactions between the substances in the sample solutions that are other than the material to be detected.

Table 3

| Sample No. | Measured Values of CEA (ng/mℓ) | | |
|---|---|---|---|
| | Reference Example | Example 3 | Comparative Example 3 |
| 21 | 186 | 186 | 754 |
| 22 | 7 | 7 | 683 |
| 23 | 5 | 4 | 241 |
| 24 | 34 | 35 | 626 |
| 25 | 359 | 358 | 565 |
| 26 | 96 | 96 | 701 |
| 27 | 57 | 58 | 703 |
| 28 | 4 | 4 | 511 |
| 29 | 3 | 4 | 487 |
| 30 | 80 | 80 | 599 |

Example 4: Measurement of Human IgG

(1) Preparation of Liposomes and Introduction of an Immobilizing Functional Group:

Using 200 μℓ of 5 mM DPPC, 100 μℓ of 10 mM cholesterol, and 1 mM of DPPE, a liposome suspension which encloses CF was prepared in a manner similar to item (2) of Example 1. Next, 0.05% glutaraldehyde solution diluted with HBS to equal amount to that of the liposome suspension was added thereto, and the mixture was left at 4°C to react overnight to obtain a liposome suspension to which is introduced the aldehyde group. Unreacted glutaraldehyde was removed by repeating several times a procedure of centrifuging at 4°C and 15,000 rpm for 20 minutes. The liposomes were suspended again with the concentration at the time of its preparation.

(2) Preparation of Liposomes with an Immobilized Anti-human IgG Antibody and the Blocking of the Immobilized Functional Group:

A liposome sediment obtained by centrifuging 1 mℓ of the liposome suspension of item (1) above at 4°C and 15,000 rpm of 20 minutes, and 1 mℓ of anti-human IgG antibody solution that has 2 mg of proteins/mℓ, were mixed, and the reaction vessel was flashed with nitrogen. Then, the system was tightly sealed and reacted overnight at 20°C with gentle shaking. Next, using a 0.5 M glycine buffer solution (pH 8.0), the above mixture was centrifuged for 20 minutes at 4°C and 15,000 rpm, and then washed. While keeping the liposomes suspended in a 0.5 M glycine buffer solution, the mixture was reacted overnight at 20°C with gentle shaking. By virtue of this reaction, the remaining immobilizing functional group remaining on the surface of the liposomes for immobilizing anti-human IgG antibody were blocked through bonding with glycine and the aldehyde group. The liposomes were used as a reagent by resuspending in $GVB^-$.

(3) Measurement of Human IgG Concentration by the Use of a Liposome Reagent with Immobilized Anti-human IgG Antibody:

Five microliters of a liposome reagent which was diluted 10-fold in advance with $GVB^+$ and 25 μℓ of a complement ($5CH_{50}$) were added to 25 μℓ of a human IgG solution having its concentration varied within the range of 0.1 to 20 mg/mℓ, and the mixture was allowed to react at 37°C for one hour. Next, the reaction

was interrupted by the addition of 100 µℓ of 0.01 M EDTA-Veronal buffer solution, fluorescence intensity was measured in the same way was in (6) of Example 1, and further, a calibration curve which shows the relationship between the IgG concentration and the relative fluorescence intensity was obtained. Using the calibration curve thus obtained, measurements were taken of the human IgG concentration on the serum samples (No. 31 through No. 40) of 10 patients. The result is shown in Table 4. In Table 4, values for Reference Example represent the values obtained by a latex aggregation method.

Comparative Example 4

An immunoassay reagent was prepared by carrying out all of the procedures in items (1) to (4) of Example 4 above, except for the omission of the blocking procedure for the immobilizing functional group described in item (2), and the human IgG concentration was measured for the samples identical to those of Example 4. The result is also included in Table 4.

As may be clear from Table 4, the immunoassay reagent of Example 4 gives rise to the measured values that agree quite well with the values obtained by a latex aggregation method. By contrast, in the case of the immunoassay reagent of Comparative Example 4, there occurred cases in which the measured value deviated substantially from that obtained by the latex aggregation method. The reason for this is that in the case of the immunoassay reagent of Comparative Example 4, non-specific reactions occurred between the substances in the sample solutions that are other than the material to be detected.

**Table 4**

| Sample No. | Measured Values of IgG (mg/mℓ) | | |
|---|---|---|---|
| | Reference Example | Example 4 | Comparative Example 4 |
| 31 | 8 | 8 | 9 |
| 32 | 11 | 12 | 14 |
| 33 | 6 | 6 | 9 |
| 34 | 17 | 16 | 18 |
| 34 | 12 | 12 | 12 |
| 36 | 8 | 8 | 13 |
| 37 | 5 | 5 | 14 |
| 38 | 12 | 11 | 14 |
| 39 | 14 | 14 | 17 |
| 40 | 19 | 18 | 26 |

Example 5: Measurement of Human IgG

(1) Preparation of Liposomes and Introduction of Immobilizing Functional Gourp:

A liposome suspension which encloses CF was prepared in a manner similar to item (2) of Example 1, by using 200 µℓ of 5 mM DPPC, 100 µℓ of 10 mM cholesterol, and 1mM of DPPE. Next, 1 mM DTBP (Pierce Chemical Co.) solution which was diluted with 0.01 M borate buffered saline (pH 8.0), in the amount equal to that of the liposome suspension, was added thereto, to prepare to liposome suspension to which is introduced imide ester

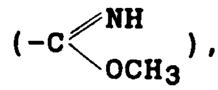

by allowing the above mixture to react at 20°C for 3 hours. Unreacted DTBP was washed by repeated centrifugation using HBS.

(2) Preparation of Liposomes with Immobilized Anti-human IgG Antibody and Blocking of the Residual Immobilizing Functional Group:

After mixing the liposome sediment obtained by centrifuged operation 1 mℓ of the liposome suspension obtained in (1) above at 4°C and 15,000 rpm for 20 minutes, and 1 mℓ of an anti-human IgG antibody solution that contains 2 mg of proteins/mℓ flashing the reaction vessel with nitrogen, the system was sealed tightly and allowed to react for 5 hours at 20°C while being kept shaken gently. Next, the mixture was washed by repeating three times the centrifugal procedure at 4°C and 15,000 rpm for 20 minutes using a 0.5 M glycine buffer solution (pH 8.0).

By the washing with the glycine buffer solution, the residual immobilization functional group (imide ester) was satisfactorily blocked. The liposome was used as a reagent by resuspending it in $GVB^-$.

(3) Measurement of Human IgG Concentration by the Use of a Liposome Reagent with Immobilized Anti-human IgG Antibody:

To 25 μℓ of a human IgG solution whose concentration was varied in the range of 0.1 to 10 mg/mℓ there were added 5 μℓ of the liposome reagent which was diluted 10-fold with $GVB^-$ and 25 μℓ of a complement (5 $CH_{50}$), and the mixture was allowed to react at 37°C for one hour. Next, the reaction was interrupted by the addition of 100 μℓ of 0.01 M EDTA-Veronal buffer solution. Then, the fluorescence intensity was measured in a manner similar to (6) of Example 1, and further, there was obtained a calibration curve which shows the relationship between the human IgG concentration and the relative fluorescence intensity. Using the calibration curve thus obtained, measurements were taken of the human IgG concentration on the serum samples (No. 41 through No. 50) of 10 patients. The result is shown in Table 5. In Table 5, values for Reference Example represent the values measured by a latex aggregation method.

Comparative Example 5

An immunoassay reagent was prepared by carrying out procedures identical to those of Example 5, except for the omission, among the procedures (1) to (4) above, of the blocking of the immobilizing functional group of item (2), and the human IgG concentration was measured for the samples identical to those of Example 5. The result is shown in Table 5.

As may be seen from Table 5, use of the immunoassay reagent of Example 5 gives measured values which agree quite well with those obtained by a latex aggregation method. In contrast, in the ace of the immunoassay reagent of Comparative Example 5, there are cases in which the measured values deviate substantially from those of a latex aggregation method. The reason for this is that in the case of the immunoassay reagent of Comparative Example 5, there are generated nonspecific reactions between substances in the sample solutions that are different from the material to be detected.

## Table 5

| Sample No. | Measured Values of IgG (Mg/mℓ) | | |
|---|---|---|---|
| | Reference Example | Example 5 | Comparative Example 5 |
| 41 | 7 | 7 | 30 |
| 42 | 32 | 32 | 40 |
| 43 | 29 | 30 | 63 |
| 44 | 28 | 29 | 72 |
| 45 | 30 | 30 | 38 |
| 46 | 3 | 3 | 6 |
| 47 | 61 | 61 | 61 |
| 48 | 55 | 55 | 58 |
| 49 | 12 | 12 | 35 |
| 50 | 18 | 8 | 19 |

Example 6: Measurement of Human Ferritin

(1) Preparation of Functional Phospholipid and Preparation of Liposome:

A liposome suspension was prepared by carrying out procedures that are identical to those of (1) and (2) of Example 1.

(2) Modification of an Anti-human Ferritin Antibody:

Using an anti-human ferritin antibody, the SH group was introduced by a procedure similar to item (3) of Example 1.

(3) Preparation of Liposomes with Immobilized Anti-human Ferritin Antibody and Blocking of the Immobilizing Functional Group:

The liposome sediment obtained by centrifuging 1 mℓ of the liposome suspension of item (1) above, at 4°C and 15,000 rpm for 20 minutes, was mixed with 2 mℓ of the modified anti-human ferritin antibody solution that contains 1 mg of proteins/mℓ obtained in (2) above. After flashing the reaction vessel with nitrogen, the system was tightly sealed and allowed to react at 20°C for 8 hours with gentle shaking.

Next, unreacted antibodies were removed by washing with $GVB^-$. In order to eliminate the residual immobilizing functional group, 2 mℓ of 1 mM cysteine solution diluted with HBS (pH 7.8) was mixed with 0.1 g of the liposome sediment. After flashing the reaction vessel with argon, the system was tightly sealed and allowed to react at 20°C for 6 hours with gentle shaking. Subsequently, unreacted cysteine was removed by washing the liposomes with $GVB^-$.

After addition of 2 mℓ of $GVB^-$ and 20 μℓ of 10% $NaN_3$ to the liposome reagent thus obtained and a thorough stirring, the system was flashed with nitrogen stored at 4°C.

(4) Measurement of Human Ferritin Concentration by the Use of a Liposome Reagent with Immobilized Anti-human Ferritin Antiobdy:

Employing a sandwich assay which makes use of isolated anti-human ferritin antibodies of rabbit, the

relationship between the human ferritin concentration and the relative fluorescence intensity was studied in a manner similar to items (6) and (7) of Example 1, to obtain a calibration curve. With the calibration curve thus obtained, the human ferritin concentration was measured for the serum samples (No. 51 through No. 60) of 10 patients. The results are shown in Table 6. The values for Reference Example in Table 6 represent the measured values obtained by RIA.

Comparative Example 6

An immunoassay reagent was prepared by carrying out the procedures, namely, items (1) to (4) above, that are identical to those of Example 6, except for the procedure of modifying the immobilizing functional group of (3). With a calibration curve for the human ferritin concentration that was obtained by the use of the reagent in the above, the human ferritin was measured for the samples that are identical to those of Example 6. The result is shown in Table 6.

As may be seen from Table 6, use of the immunoassay reagent of Example 6 gave rise to the measured values that agree quite well with those due to RIA. In contrast, in the case of the immunoassays reagent of Comparative Example 6, measurements were not possible to be taken. The reason for this is that nonspecific reactions are being generated in the case of the immunoassay reagent of Comparative Example 6.

## Table 6

| Sample No. | Measured Values of Ferritin (ng/ml) | | |
|---|---|---|---|
| | Reference Example | Example 6 | Comparative Example 6 |
| 51 | 450 | 450 | 1867 |
| 52 | 730 | 730 | 2382 |
| 53 | 332 | 332 | 2163 |
| 54 | 911 | 911 | 1911 |
| 55 | 990 | 1001 | 1966 |
| 56 | 319 | 318 | 2415 |
| 57 | 117 | 151 | 2211 |
| 58 | 35 | 31 | 967 |
| 59 | 67 | 67 | 1446 |
| 60 | 519 | 519 | 1987 |

**Claims**

1. An immunoassay reagent comprising liposomes which are composed of (1) a phospholipid or a glycolipid, (2) a marker material enclosed in the said liposomes, and (3) an antigen, an antibody, a part of the antibody or a combination thereof immobilized on the said liposomes' surface through a cross-linking agent comprising functional groups, characterized in that said functional groups remaining unreacted on the liposome surface after immobilization of the antigen, the antibody, a part of the antibody or a combination thereof on said liposomes, are chemically modified by a blocking agent.

2. The reagent according to claim 1, characterized in that said blocking agent is a compound containing an SH group.

3. The reagent according to claim 1, characterized in that said blocking agent is a compound containing an amino group.

4. The reagent according to claim 2, characterized in that said blocking agent comprises cysteine or dithiothreitol.

5. The reagent according to claim 3, characterized in that said blocking agent comprises glycine or TRIS.

6. The reagent according to claim 1, characterized in that said liposomes further comprise cholesterol.

7. The reagent according to claim 6 characterized in that the molar ratio of said cholesterol to said phospholipid or glycolipid is between 0.1 and 5.0.

8. The reagent according to claim 1, characterized in that said cross-linking agent comprises N-succinimidyl-3-(2-pyridyldithio)propionate, disuccinimidyl suberate, 3,3'-dithiobis-(sulfosuccinimidylpropionate), dimethyl adipimidate•2HCl, dimethyl-3,3'-dithiobispropionimidate•2HCl, an aliphatic dialdehyde or a hypo-acidic chloride.

9. The reagent according to claim 1, characterized in that said marker material is a fluorescent compound, a luminous compound, an absorptive compound, a saccharide, an ionic compound, an enzyme, a coenzyme or a radical compound.

10. The reagent according to claim 1, characterized in that said antigen, antibody, or a part of antibody comprise a protein, a peptide, or a glycoprotein.

11. The reagent according to claim 1, characterized in that the amount of phospholipid or glycolipid which has reacted with said cross-linking agent is within the range of 0.01 to 30 mol%.

12. The reagent according to claim 1, characterized in that the concentration of the antigen, the antibody, or part of the antibody which is immobilized, for liposomes having a lipid equivalent of 0.5 mM, is within the range of 0.01 to 20 mg/mℓ.

13. The reagent according to claim 1, characterized in that said phospholipid comprises a $C_{12-18}$ fatty acid.

14. The reagent according to claim 1, characterized in that said glycolipid comprises a $C_{12-18}$ fatty acid.

15. A method for assaying a substance in a sample, comprising:
(i) adding to said sample an immunoassay reagent comprising liposomes which are composed of (1) a phospholipid or a glycolipid, (2) a marker material enclosed in said liposomes, and (3) an antigen, an antibody, a part of the antibody or a combination thereof immobilized on said liposomes through a cross-linking agent comprising functional groups,
characterized in that said functional groups remaining unreacted on the liposome surface after immobilization of the antigen, the antibody, a part of the antibody or a combination thereof on said liposomes, are chemically modified by a blocking agent,
(ii) analyzing the amount of marker material released from the said liposomes by the action of complement.

16. The method according to claim 15, characterized in that said antigen, antibody or a part of antibody comprises h-fetoprotein, basic fetoprotein, carcinoembyronic antigen, pancreatic oncofetal antigen, an immunoglobulin, a peptide hormone or a drug.

**Patentansprüche**

1. Immuntestreagens, umfassend Liposomen, die aus
(1) einem Phospholipid oder einem Glycolipid,
(2) einem in den Liposomen eingeschlossenen Markermaterial und
(3) einem Antigen, einem Antikörper, einem Teil des Antikörpers oder einer Kombination derselben

in auf der Liposomenoberfläche durch ein Vernetzungsmittel mit funktionellen Gruppen immobilisierter Form bestehen,
**dadurch gekennzeichnet,** daß die nach Immobilisierung des Antigens, des Antikörpers, eines Teils des Antikörpers oder einer Kombination derselben auf den Liposomen auf der Liposomoberfläche unumgesetzt verbliebenen funktionellen Gruppen durch ein Blockiermittel chemisch modifiziert sind.

**2.** Reagens nach Anspruch 1, dadurch gekennzeichnet, daß das Blockiermittel aus einer Verbindung mit einer SH-Gruppe besteht.

**3.** Reagens nach Anspruch 1, dadurch gekennzeichnet, daß das Blockiermittel aus einer Verbindung mit einer Aminogruppe besteht.

**4.** Reagens nach Anspruch 2, dadurch gekennzeichnet, daß das Blockiermittel aus Cystein oder Dithiothreit besteht.

**5.** Reagens nach Anspruch 3, dadurch gekennzeichnet, daß das Blockiermittel aus Glycin oder TRIS besteht.

**6.** Reagens nach Anspruch 1, dadurch gekennzeichnet, daß die Liposomen zusätzlich Cholesterin enthalten.

**7.** Reagens nach Anspruch 6, dadurch gekennzeichnet, daß das Molverhältnis Cholesterin/Phospholipid oder Glycolipid zwischen 0,1 und 5,0 liegt.

**8.** Reagens nach Anspruch 1, dadurch gekennzeichnet, daß das Vernetzungsmittel aus N-Succinimidyl-3-(2-pyridyldithio)-propionat, Disuccinimidylsuberat, 3,3'-Dithiobis-(sulfosuccinimidylpropionat), Dimethyladipimidat•2HCl, Dimethyl-3,3'-dithiobispropionimidat•2HCl, einem aliphatischen Dialdehyd oder einem hyposauren Chlorid besteht.

**9.** Reagens nach Anspruch 1, dadurch gekennzeichnet, daß das Markermaterial aus einer fluoreszierenden Verbindung, einer leuchtenden Verbindung, einer absorptiven Verbindung, einem Saccharid, einer ionischen Verbindung, einem Enzym, einem Coenzym oder einer radikalischen Verbindung besteht.

**10.** Reagens nach Anspruch 1, dadurch gekennzeichnet, daß das Antigen, der Antikörper oder ein Teil des Antikörpers aus einem Protein, einem Peptid oder einem Glycoprotein besteht.

**11.** Reagens nach Anspruch 1, dadurch gekennzeichnet, daß die Menge an Phospholipid oder Glycolipid, die mit dem Vernetzungsmittel reagiert hat, im Bereich von 0,01 - 30 Mol-% liegt.

**12.** Reagens nach Anspruch 1, dadurch gekennzeichnet, daß die Konzentration des Antigens, des Antikörpers oder eines Teils des Antikörpers, das bzw. der immobilisiert wurde, für Liposomen eines Lipidäquivalents von 0,5 mM im Bereich von 0,01 - 20 mg/ml liegt.

**13.** Reagens nach Anspruch 1, dadurch gekennzeichnet, daß das Phospholipid eine $C_{12-18}$-Fettsäure enthält.

**14.** Reagens nach Anspruch 1, dadurch gekennzeichnet, daß das Glycolipid eine $C_{12-18}$-Fettsäure enthält.

**15.** Verfahren zum Testen einer Substanz in einer Probe durch
(i) Zugabe eines Immuntestreagenses, umfassend Liposomen, die aus
(1) einem Phospholipid oder einem Glycolipid,
(2) einem in den Liposomen eingeschlossenen Markermaterial und
(3) einem Antigen, einen Antikörper, einem Teil des Antikörpers oder einer Kombination derselben in auf den Liposomen durch ein Vernetzungsmittel mit funktionellen Gruppen immobilisierter Form bestehen, zu der Probe, wobei die nach Immobilisierung des Antigens, des Antikörpers, eines Teils des Antikörpers oder einer Kombination derselben auf den Liposomen auf der Liposomoberfläche unumgesetzt verbliebenen funktionellen Gruppen durch ein Blockiermittel chemisch modifiziert sind, und

(ii) Analysieren der Menge an aus den Liposomem durch die Komplementwirkung freigesetztem Markermaterial.

**16.** Verfahren nach Anspruch 15, dadurch gekennzeichnet, daß das Antigen, der Antikörper oder ein Teil des Antikörpers h-Fetoprotein, basisches Fetoprotein, carcinoembyronisches Antigen, pankreatisches onkofetales Antigen, ein Inmunglobulin, ein Peptidhormon oder ein Arzneimittel umfaßt.

**Revendications**

**1.** Dans un réactif de dosage immunologique comprenant des liposomes qui sont composés de (1) un phospholipide ou un glycolipide, (2) un marqueur enfermé dans lesdits liposomes et (3) un antigène, un anticorps, une partie de l'anticorps ou une de leurs combinaisons, immobilisés sur la surface desdits liposomes au moyen d'un agent réticulant comprenant des groupes fonctionnels, caractérisé en ce que lesdits groupes fonctionnels n'ayant pas réagi restant sur la surface des liposomesaprès immobilisation de l'oxygène, de l'anticorps, d'une partie de l'anticorps ou de leur combinaison sur lesdits liposomes sont modifiés chimiquement par un agent bloquant.

**2.** Le réactif selon la revendication 1, caractérisé en ce que ledit agent bloquant est un composé contenant un groupe SH.

**3.** Le réactif selon la revendication 1, caractérisé en ce que ledit agent bloquant est un composé contenant un groupe amino.

**4.** Le réactif selon la revendication 2, caractérisé en ce que ledit agent bloquant de la cystéine ou du dithiothréitol.

**5.** Le réactif selon la revendication 3, caractérisé en ce que ledit agent bloquant comprend de la glycine ou du TRIS.

**6.** Le réactif selon la revendication 1, caractérisé en ce que lesdits liposomes comprennent en outre du cholestérol.

**7.** Le réactif selon la revendication 6, caractérisé en ce que le rapport molaire dudit cholestérol audit phospholipide ou glycolipide est compris entre 0,1 et 5,0.

**8.** Le réaction selon la revendication 1, caractérisé en ce que ledit agent réticulant comprend du 3-(2-pyridyldithio)propionate de N-succinimidyle, du subérate de disuccinimidyle, du dipropionate de 3,3'-dithiobis(sulfosuccinimidyle), du dichlorhydrate d'adipimidate de diméthyle, du dichlorhydrate de 3,3'-dithiobispropionimidate de diméthyle, un dialdéhyde aliphatique ou un chlorure d'hypo-acide.

**9.** Le réactif selon la revendication 1, caractérisé en ce que ledit marqueur est un composé fluorescent, un composé luminescent un composé absorbant, un saccharide, un composé ionique, une enzyme, une coenzyme ou un composé radicalaire.

**10.** Le réactif selon la revendication 1, caractérisé en ce que ledit antigène, ledit anticorps ou une partie dudit anticorps comprennent une protéine, un peptide ou une glycoprotéine.

**11.** Le réactif selon la revendication 1, caractérisé en ce que la quantité de phospholipide ou de glycolipide qui a réagi avec ledit agent réticulant est dans la gamme de 0,01 à 30 mol %.

**12.** Le réactif selon la revendication 1, caractérisé en ce que la concentration de l'antigène, de l'anticorps, ou d'une partie de l'anticorps qui est immobilisé pour les liposomes ayant un équivalent de lipide de 0,5 mM, est dans la gamme de 0,01 à 20 mg/ml.

**13.** Le réactif selon la revendication 1, caractérisé en ce que ledit phospholipide comprend un acide gras en $C_{12}$-$C_{18}$.

**14.** Le réactif selon la revendication 1, caractérisé en ce que ledit glycolipide comprend un acide gras en

$C_{12}$-$C_{18}$.

**15.** Un procédé pour déterminer une substance dans un échantillon, comprenant les étapes suivantes :
(i) on ajoute audit échantillon un réactif de dosage immunologique comprenant des liposomes qui sont composés de (1) un phospholipide ou un glycolipide, (2) un marqueur enfermé dans lesdits liposomes et (3) un antigène, un anticorps, une partie de l'anticorps ou une de leurs combinaisons, immobilisés sur lesdits liposomes par un agent réticulant comprenant des groupes fonctionnels, caractérisé en ce que lesdits groupes fonctionnels n'ayant pas réagi restant sur la surface des liposomes après immobilisation de l'antigène, de l'anticorps, d'une partie de l'anticorps ou d'une de leurs combinaisons sur lesdits liposomes sont chimiquement modifiés par un agent bloquant,
(ii) on analyse la quantité de marqueur libéré par lesdits liposomes par l'action du complément.

**16.** Le procédé selon la revendication 15, caractérisé en ce que ledit antigène, ledit anticorps ou une partie dudit anticorps comprend une h-foetoprotéine, une foetoprotéine basique, un antigène carcinomembryonaire, un antigène oncofoetal pancréatique, une immunoglobuline, une hormone peptidique ou un médicament.

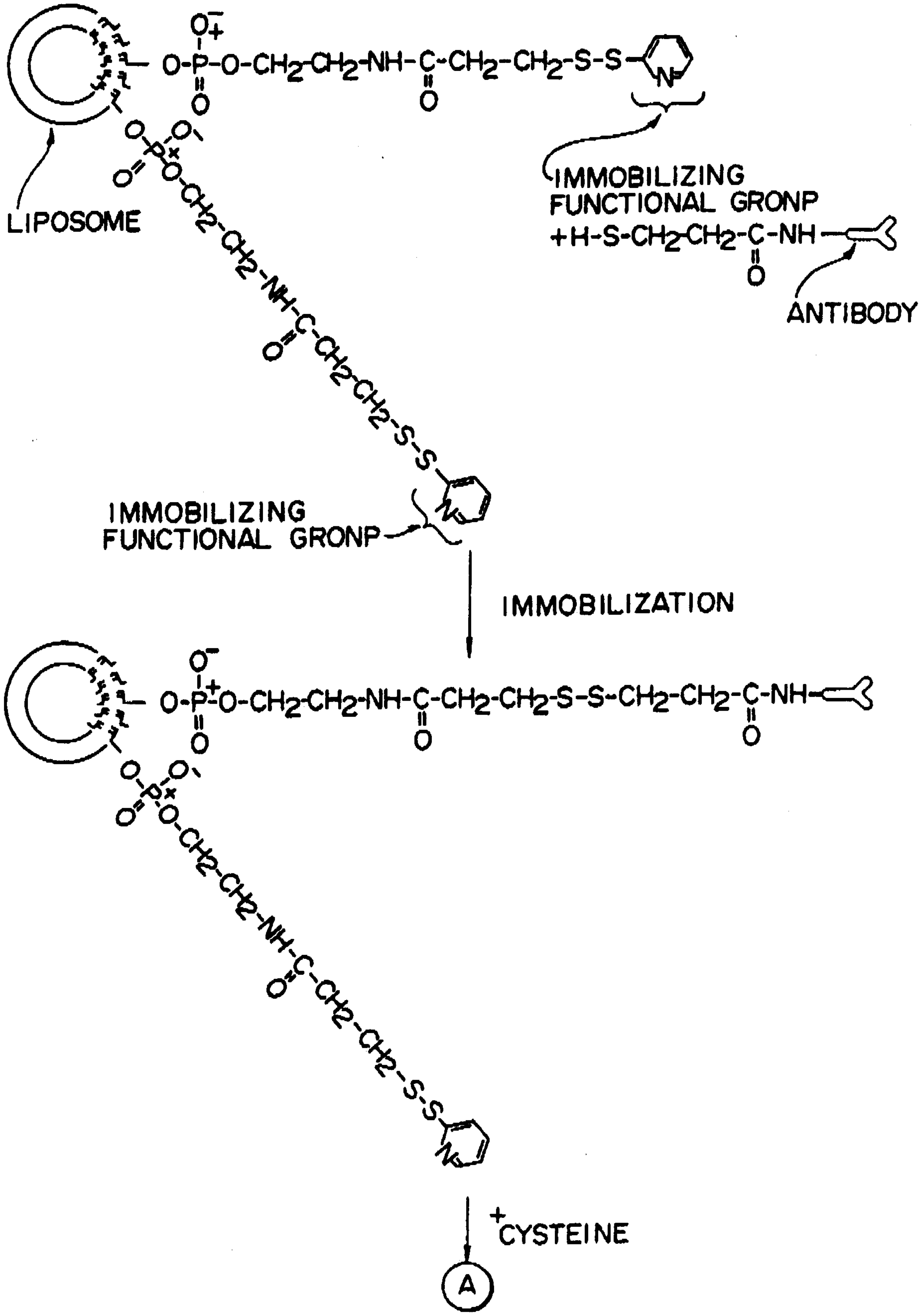
LIPOSOME
IMMOBILIZING FUNCTIONAL GRONP
ANTIBODY
IMMOBILIZING FUNCTIONAL GRONP
IMMOBILIZATION
+CYSTEINE

F I G. 1A

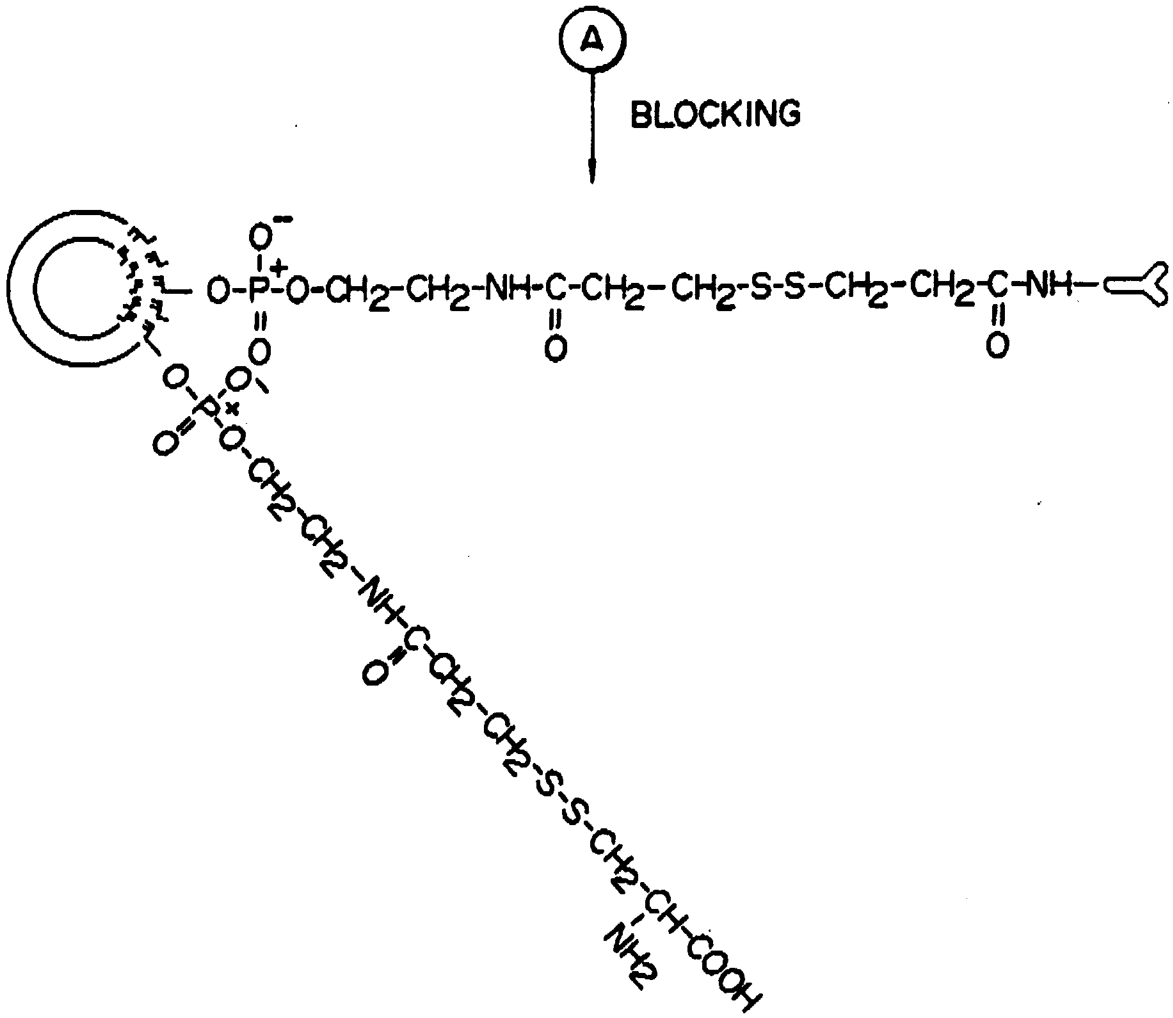


BLOCKING SCHEME USING A SUBSTANCE CONTAINING SH GRONP (CYSTEINE)

F I G. 1B

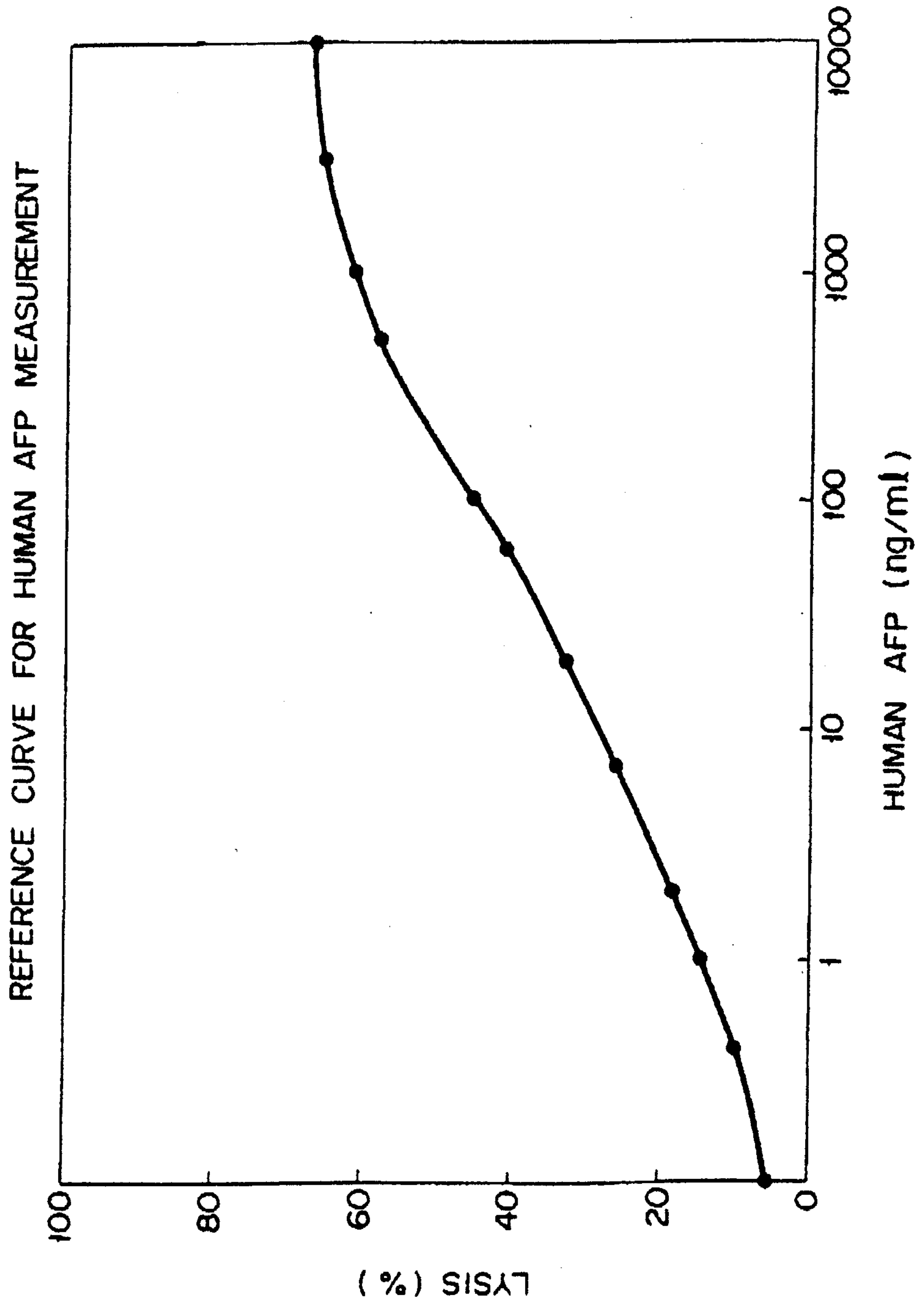
REFERENCE CURVE FOR HUMAN AFP MEASUREMENT
LYSIS (%)
0
20
40
60
80
100
1
10
100
1000
10000
HUMAN AFP (ng/ml)

F I G. 2